# EUROPEAN PATENT APPLICATION

(11) **EP 4 623 853 A1**
(43) Date of publication of application: **01.10.2025**
(21) Application number: 25165893.6
(22) Date of filing: 25.03.2025
(51) Int. Cl.: A61B 50/31, A61N 1/39, A61N 1/375

(54) **MEDICAL DEVICE WITH ENHANCED STORAGE AND PORTABILITY**

(30) Priority: 25.03.2024 US 202463569388 P; 21.03.2025 US 202519087118
(71) Applicant: Stryker Corporation, Portage, MI 49002-9711 (US)
(72) Inventor: WEGMANN, Brittany Kendra, Portage, 49002 (US); WONG, Jeremy, Portage, 49002 (US); ALVIAR, Christopher G., Portage, 49002 (US)
(74) Representative: V.O.

(57) **Abstract**

An example modular bag system includes a bracket configured to be removably coupled to a housing of a medical device and a bag removably coupled to a first arm of the bracket. The first arm extends in a first direction and configured to conform to a portion of a first side of the medical device housing. The bracket also includes a second arm extending in a second direction and configured to conform to a portion of a second side of the medical device housing. A foot extends from the second arm.

## Description

### CROSS-REFERENCE TO RELATED APPLICATION

This application claims priority to U.S. Provisional App. No. 63/569,388, which was filed on March 25, 2024 and is hereby incorporated by reference herein in its entirety.

### BACKGROUND

Some medical devices are utilized in non-clinical environments. For example, emergency medical services (EMS) personnel may be deployed to a rescue scene in which a patient has exhibited sudden, and potentially dangerous, symptoms. An EMS provider, for instance, utilizes a portable monitor-defibrillator to assess the condition of the patient and to potentially administer a treatment before the patient is transported to a hospital. In some cases, the EMS provider must physically carry the monitor-defibrillator to the location of the patient.

### BRIEF DESCRIPTION OF THE DRAWINGS

FIG. 1 illustrates an example environment of a medical device with enhanced storage and portability features.
FIGS. 2A, 2B, 2C, and 2D illustrate examples of modular storage systems that can be utilized to store accessories of medical devices. FIG. 2A illustrates a first bag mounted on a first bracket. FIG. 2B illustrates a second bag mounted on a second bracket. FIG. 2C illustrates a third bag mounted on a third bracket. FIG. 2D illustrates a fourth bag mounted on a fourth bracket.
FIG. 3 illustrates an example of multiple bags mounted on a medical device.
FIGS. 4A and 4B illustrate a process for injection molding a housing that can accommodate an adapter without having an undercut. FIG. 4A illustrates a mold that is configured to be injected with a molding material. FIG. 4B illustrates how the adapter can be inserted into the housing.
FIG. 5 illustrates an example of an adapter.
FIG. 6 illustrates an example process for switching an adapter from a first medical device to a second medical device.
FIG. 7 illustrates an example of an external defibrillator configured to perform various functions described herein.

### DETAILED DESCRIPTION

Various implementations described herein relate to techniques for enhancing the usability and portability of medical devices. In some implementations, a modular storage solution provides efficient access to accessories of a portable medical device during deployment at a rescue scene. In some examples, a bracket is attached to a bag configured to store a medical device accessory as well as to the housing of the medical device itself. In some examples, the bracket is attached to multiple sides of the medical device, and can provide structural support for the medical device. The bracket, for instance, can be removably coupled with the housing of the medical device. In various cases, the bag includes a rigid compartment as well as a flap that hangs over an opening to the bag. Accordingly, the bag prevents contamination of one or more accessory devices disposed inside of the bag. In various cases, an accessory is removably coupled with a port of the medical device during storage. For example, a cable may extend from the port of the medical device into the bag with the accessory device before the medical device is utilized.

In some implementations, a fastener is used for connecting bags, shoulder straps, and other elements to the medical device. The fastener, for instance, can be disposed inside of the housing of the medical device. In various cases, the fastener accommodates a quick-detach (QD) adapter. In some examples, the fastener is welded to the housing of the medical device, which does not include an undercut. Accordingly, the housing may be designed to accommodate the fastener and to be efficiently manufactured using injection molding. In some cases, the fastener is disposed inside of a bracket that is attached to an accessory bag. According to some cases, multiple different types of medical devices may utilize dimensionally equivalent fasteners, such that a shoulder strap can be efficiently attached to the different types of medical devices in a rescue environment.

Various implementations will now be described with reference to the accompanying figures.

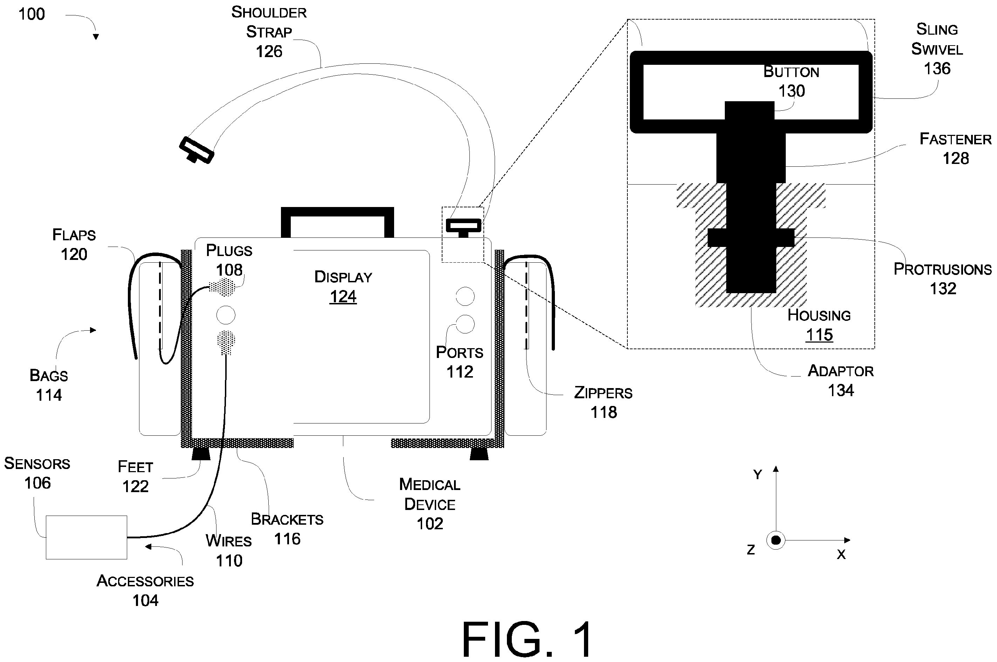

FIG. 1 illustrates an example environment 100 of a medical device 102 with enhanced storage and portability features. The term "medical device," and its equivalents, may refer to a device that is configured to detect a physiological parameter of a subject (e.g., a human), predict a condition of the subject, report the physiological parameter and/or predicted condition, administer a treatment to the subject, or any combination thereof. The terms "treatment," "therapy," and their equivalents, may refer to an attempted remediation of a health condition. A treatment, for instance, can include administration of a signal to a subject (e.g., administration of an electrical shock to the heart of the subject), administration of a substance to the subject, administration of a force to the subject (e.g., administration of chest compressions), or any combination thereof. The terms "physiological parameter," "parameter," and their equivalents, may refer to a metric that is indicative of a condition of a subject's body. Examples of physiological parameters include, for instance, an electrocardiogram (ECG), a blood pressure, a blood oxygenation (e.g., regional oxygenation, pulse oxygenation, plethysmograph, etc.), an airway parameter (e.g., a capnograph, a partial pressure of CO2, a partial pressure of O2, a tidal volume, flow rate through an airway, airway pressure, respiration rate, etc.), a temperature (e.g., a core temperature, a temperature of an extremity, etc.), a blood flow (e.g., a blood velocity, blood flow rate, etc.), a heart rate, a pulse rate, a motion (e.g., an acceleration), a concentration of a chemical in a body fluid (e.g., blood glucose), or any combination thereof.

In various cases, the medical device 102 is a portable medical device. The term "portable," and its equivalents, may refer to a device that can operate without being connected to mains power and is configured to be moved (e.g., carried) by a single operator. For instance, the medical device 102 may include an onboard power source that supplies electrical energy to one or more circuits within the medical device 102 during operation. Examples of the power source include, for instance, a battery, a capacitor, a wireless charger (e.g., an antenna), a dynamo, a solar panel, or any combination thereof. For example, the medical device 102 may include a rechargeable battery. In various cases, the medical device 102 includes a handle that enables a single user to carry the medical device 102 from a storage area (e.g., an ambulance) to a patient in medical distress at a rescue scene.

The medical device 102 is configured to be coupled to various accessories 104. The term "accessory," and its equivalents, may refer to a device that, when coupled with a base device, enables the base device to perform one or more functions via the accessory. For instance, the accessories 104 may include sensors 106 that detect one or more physiological parameters of a subject and output, to the medical device 102, data indicating the physiological parameter(s). Examples of the sensors 106 include one or more electrodes, a blood pressure sensor (e.g., a catheter-based sensor, a blood pressure cuff, an ultrasound-based blood pressure sensor, etc.), an oxygenation sensor (e.g., an oximeter), a pressure sensor, a flow sensor, a gas sensor, a blood flow sensor (e.g., an ultrasound transducer configured to perform Doppler-based measurements on blood flow), an accelerometer, a gyroscope, a chemical sensor, or any combination thereof. In various examples, the accessories 104 are configured to administer a treatment to a subject. For instance, the accessories 104 may include defibrillation electrodes, laryngoscopes, ventilation devices, chest compression devices (e.g., a band or plunger configured to administer chest compressions), or any combination thereof. In some cases, the accessories 104 include other elements, such as printers, recording devices, external display devices, and the like.

The accessories 104 are configured to be removably coupled with the medical device 102. The term "removably coupled," and its equivalents, may refer to two elements that can be manually connected to one another and removed from one another. In various cases, the accessories 104 are configured to be communicatively and/or electrically coupled with the medical device 102. In particular, the accessories 104 include plugs 108 that are connected to the sensors 106 via wires 110. The term "plug," and its equivalents, may refer to a connector that is configured to establish an electrical connection with a port when the connector is physically mated with the port. The terms "wire," "cable," and their equivalents, may refer to an elongated and flexible structure over which signals can be transmitted. For instance, a wire may include an electrically conductive (e.g., metal) core surrounded by an electrically insulative cover (e.g., a woven and/or polymer cover). The plugs 108 are configured to be physically coupled to various ports 112 of the medical device 102. The term "port," and its equivalents, may refer to a hollow receptacle configured to receive a plug and establish an electrical connection with the plug. In various cases, the ports 112 of the medical device 102 have different shapes. For instance, only one type of accessory 104 may be connected to an example port 112 of the medical device 102.

In various cases, the medical device 102 is designed to provide monitoring and care to a patient in a non-clinical environment. In various cases, the patient is in a pre-clinical environment, and the medical device 102 is utilized to monitor and/or treat the patient before the patient can be transported to a clinical setting, such as a hospital. Thus, the medical device 102 may be utilized in sudden medical emergencies, such as cardiac arrest, where delays in care can have significant consequences to the care of the patient. In particular cases, it may be beneficial to quickly attach the accessories 104 to the patient in the rescue setting.

Rapidly deploying the medical device 102 in a remote environment, however, can be challenging. In various cases, it may be beneficial to store the accessories 104 with the medical device 102, such that a single rescuer can easily and simultaneously carry the medical device 102 and accessories 104 together. In some cases, the accessories 104 may be stored in a bag that is attached to the medical device 102. However, in these cases, the wires 110 and plugs 108 may be tangled together during storage. The process of detangling the wires 110 and attaching the plugs 108 to the medical device can result in dangerous delays to patient monitoring and treatments. Moreover, attaching the plugs 108 to the ports 112 may cause additional delays. Therefore, it may be beneficial to prevent tangling of the wires 110, store the accessories 104 while they are plugged into the ports 112, and provide any other mechanism that can speed rapid deployment of the medical device 102 at the rescue scene.

In various implementations of the present disclosure, a modular storage system is utilized to store the accessories 104 and other essential items before the medical device 102 is deployed and used to monitor or treat a patient. The modular storage system, for instance, includes multiple bags 114. The multiple bags 114, for instance, are configured to store the accessories 104 between uses of the medical device 102.

The bags 114 are attached to a housing 115 of the medical device 102 by brackets 116. The term "housing," and its equivalents, may refer to a physical outer layer of a device, which may prevent internal components of the device from being exposed to moisture, dust, and other contaminants. In various cases, the housing 115 of the medical device includes a metal (e.g., stainless steel) and/or a polymer, such as acrylonitrile butadiene styrene (ABS), acrylic poly methyl methacrylate (PMMA), polycarbonate, polypropylene, polyethylene, polyamide, polystyrene, polymethyl methacrylate, polyethylene terephthalate, silicone, or any combination thereof. For instance, at least a portion of the housing 115 may be manufactured via an injection molding technique.

The bags 114, in various cases, have one or more features that protect the accessories 104 during storage. In various cases, the bags 114 include one or more rigid compartments. The term "rigid," and its equivalents, may refer to an inflexible material that resists deformation when a force is applied. In some cases, a rigid compartment includes a rigid material, such as a metal or a polymer. Optionally, a rigid compartment includes a flexible outer covering, such as a woven material or a flexible polymer. Although not specifically pictured in FIG. 1, in some cases, the interior of one or more of the bags 114 may be subdivided by dividing structures that extend from one side of the interior to another side of the interior. Thus, one or more of the bags 114 may include multiple internal compartments.

The bags 114, in some cases, include zippers 118. Each zipper 118, for instance, includes a movable slider that traverses a chain with two sides that are physically pressed and bound together when traversed by the movable slider. In some cases, an individual zipper 118 includes multiple movable sliders. According to various cases, the two sides of the chain are coupled to flexible portions of the bags 114. For instance, the two sides of the chain of an example zipper 118 may be connected to two sides of the flexible outer covering of a rigid compartment of one of the bags 114. The chain of the zipper 118, for instance, extends along a segment of the bags that is within a threshold distance (e.g., 1 cm, 2 cm, 5 cm, or the like) of the ports 112. Thus, the accessories 104 may be efficiently stored in the bags 114 while simultaneously being plugged into the ports 112 of the medical device.

In some cases, the bags 114 include flaps 120 that hang over the zippers 118. In various examples, the flaps 120 include a flexible material that is partially bound to another portion of the bags 114. For instance, the flaps 120 may be sewn to the flexible outer coverings of the bags 114. When the bags 114 and brackets 116 are bound to the medical device 102, and the medical device is held upright, a gravitational force may cause the flaps 120 to be disposed over the chains of the zippers 118. In various cases, the flaps 120 may prevent dust, bodily fluids, moisture, or other contaminants from entering the interior of the bags 114, such as when the zippers 118 are open.

The brackets 116, in various cases, are rigid structures that are configured to be bound to the housing 115 of the medical device 102. The brackets 116, for instance, may include a metal and/or a polymer. In some cases, an individual bracket 116 includes multiple portions, which may extend in different directions, such as an x direction, a y direction, or a z direction. According to some cases, the individual bracket 116 includes a connecting piece that physically couples multiple portions of the bracket 116 together. In various examples, multiple portions of the bracket 116 are shaped to mate with multiple sides of the housing 115 of the medical device. Fasteners may connect the brackets 116 to the housing 115 of the medical device 102. The term "fastener," and its equivalents, may refer to a mechanical apparatus that connects one element to another element. Examples of fasteners include screws, adhesives, nails, clips, hook-and-loop fasteners, clamps, quick release fasteners, quick detach (QD) fasteners, and the like. For example, the brackets 116 may include one or more holes configured to accommodate screws, such that the brackets 116 may be screwed into the housing 115 of the medical device 102.

In various cases, the brackets 116 enhance the structural integrity of the housing 115 of the medical device 102. For example, the presence of the brackets 116 may reduce the risk that the housing 115 will break or be damaged when the medical device 102 is dropped or otherwise makes contact with an external surface. Thus, in addition to supporting the bags 114, the brackets 116 may enhance the functionality of the medical device 102 itself.

The brackets 116, in some cases, include feet 122 (e.g., at least one foot) that are configured to support the weight of the medical device 102 and the bags 114. For instance, portions of the brackets 116 may be configured to be coupled with a bottom surface of the medical device 102 (e.g., a lower surface when the medical device 102 is in an upright position during operation). In some cases, the portions of the brackets 116 are perpendicular. In some examples, the portions of the brackets 116 are curved, such that they accommodate a curved outer surface of the medical device 102.

These portions of the brackets 116 may be disposed between the feet 122 and the bottom surface of the medical device 102. Accordingly, when the medical device 102 is resting on a horizontal surface (e.g., the ground) in an upright orientation, the feet 122 are in direct contact with the horizontal surface. In various cases, the feet 122 include a rubberized material, such as a silicone, polyisoprene, neoprene, nitrile rubber, styrene-butadiene rubber (SBR), polyisobutylene, chlorosulphonated polyethylene, or any combination thereof. In some cases, the rubberized material is compressible. According to some cases, a coefficient of friction of the feet 122 is above a threshold (e.g., 0.5, 0.6, 0.7, 0.8, 0.9, or 1.0), such that the feet 122 prevent the medical device 102 from slipping along the horizontal surface. In some cases, the feet 122 cause the medical device 102 to tilt when the medical device 102 is in an upright orientation and resting on the horizontal surface. Accordingly, a display 124 of the medical device 102 is tilted in a y direction that improves visibility from the perspective of a rescuer whose eyes are at a greater height than the medical device 102 during operation. Thus, the rescuer may more easily view information visually presented on the display 124 due to the presence of the feet 122.

Various implementations of the present disclosure relate to techniques for improving portability of the medical device 102 and the accessories 104. In some cases, a rescuer or other user may prefer to carry the medical device 102 using a shoulder strap 126 rather than a handle. In some cases, the shoulder strap 126 causes the medical device 102 to be easier to carry for the rescuer. However, in cases where the rescuer may be carrying multiple devices and equipment using different bags, it could be difficult for the rescuer to release the medical device 102 in the rescue scene if the shoulder strap 126 is permanently fixed to the housing 115 of the medical device 102.

In some implementation, the shoulder strap 126 includes a fastener 128 that can be efficiently released from the housing of the medical device 102. In various cases, the fastener 128 is a QD fastener. The terms "quick-detach," "QD," and their equivalents, may refer to a type of fastener mechanism that releases from a substrate by activation of a single mechanical apparatus, such as a button. For example, the fastener 128 includes a button 130 that, when pressed, causes protrusions 132 of the fastener 128 to retract. Internally, the fastener 128 may include one or more springs and/or gears that cause the protrusions 132 to retract when the button 130 is pressed.

When the protrusions 132 extend, the fastener 128 is configured to be attached to an adapter 134. The term "adapter," and its equivalents, may refer to an apparatus that is configured to be connected to a fastener. In various cases, the adapter 134 is disposed in the housing 115 of the medical device 102. The adapter 134, for instance, has a concave structure, such that the fastener 128 may be disposed within an interior space of the adapter 134. A rim of the interior space of the adapter 134, for instance, has a relatively narrow width, whereas another portion of the interior space of the adapter 134 may have a relatively large width. Accordingly, when the protrusions 132 of the fastener 128 are retracted, the fastener 128 may be inserted into the interior space of the adapter 134. Further, when the protrusions 132 of the fastener 128 are extended, the fastener 128 may not be removable from the adapter 134, because the width of the fastener 128 with the extended protrusions 132 may be greater than the narrow width of the rim of the adapter 134. According to various cases, the fastener 128 may be dimensionally equivalent to the interior space of the adapter 134 when the protrusions 132 are extended.

The adapter 134 is disposed in the housing 115 such that the adapter 134 does not extend from an exterior surface of the housing 115. For example, an upper surface of the adapter 134 is coplanar with an outer surface of the housing 115. Accordingly, the adapter 134 is unlikely to attach to wires or other elements in the rescue environment during use of the medical device 102.

Further, the adapter 134 is relatively simple to manufacture within the housing 115. In various cases, an outer shape of the adapter 134 fits within a space in the housing 115. The portion of the housing 115 that is disposed against the adapter 134 lacks an undercut. The term "undercut," and its equivalents, may refer to a portion of an injection-molded part that prevents ejection from the mold during manufacturing. In implementations in which the housing 115 is injection molded, due to the shape of the adapter 134, the housing 115 may be easily removed from the mold due to the lack of an undercut.

In various implementations, the fastener 128 includes a sling swivel 136 that is coupled to a band of the shoulder strap 126. The term "sling swivel," and its equivalents, may refer to a bale that is configured to be attached to a sling (e.g., a band) and is also configured to rotate. For instance, the sling swivel 136 of FIG. 1 is configured to rotate around an axis defined by the y direction. The rotation of the sling swivel 136 enables the sling swivel 136 to naturally reposition based on an amount of tension and/or position of the shoulder strap 126.

Although not specifically illustrated in FIG. 1, adapters similar to the adapter 134 can be utilized to attach other elements of the environment 100 together. For example, a fastener may be configured to connect to an adapter 134 in order to fasten the brackets 116 to the medical device 102, to fasten the bags 114 to the brackets 116, to fasten the shoulder strap 126 to the brackets 116, to connect the feet 122 to the brackets 116, or any combination thereof.

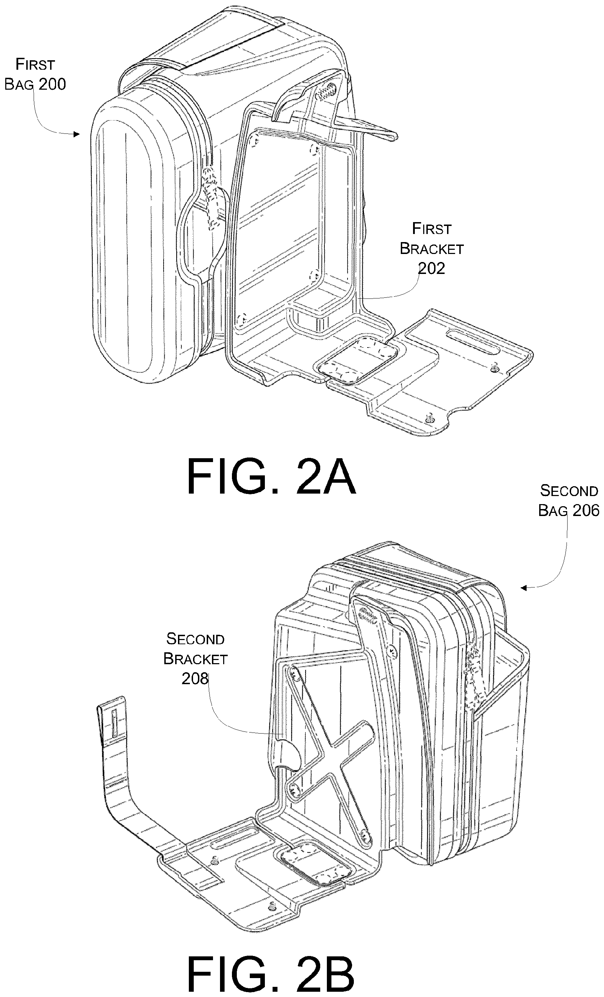

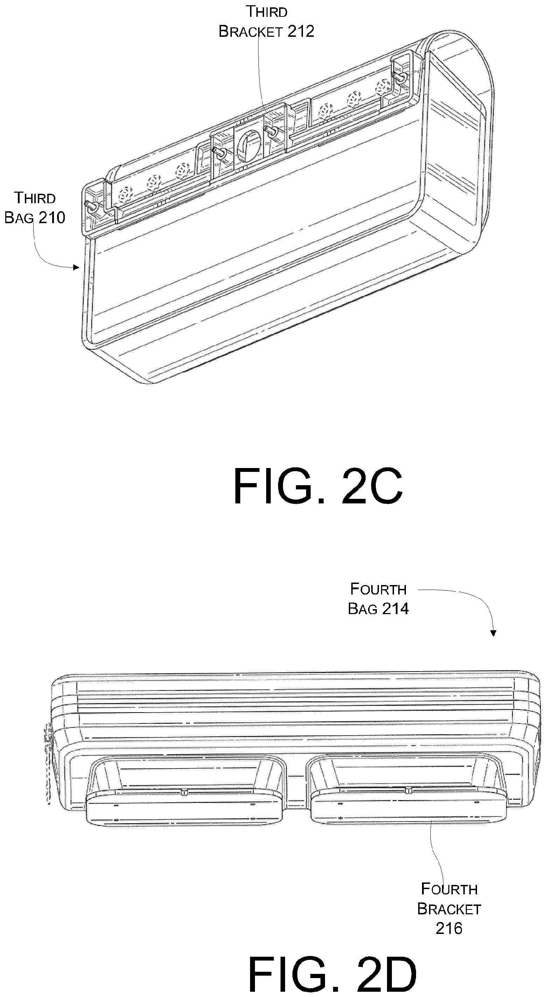

FIGS. 2A, 2B, 2C, and 2D illustrate examples of modular storage systems that can be utilized to store accessories of medical devices. FIG. 2A illustrates a first bag 200 mounted on a first bracket 202. FIG. 2B illustrates a second bag 204 mounted on a second bracket 206. FIG. 2C illustrates a third bag 208 mounted on a third bracket 210. FIG. 2D illustrates a fourth bag 212 mounted on a fourth bracket 214. In various cases, the bags 200, 204, 208, and 212 correspond to the bags 114 and the brackets 202, 206, 210, and 214 correspond to the brackets 116. In various implementations, the bags 200, 204, 208, and 212 are mounted on the same base device (e.g., a medical device) via the brackets 202, 206, 210, and 214.

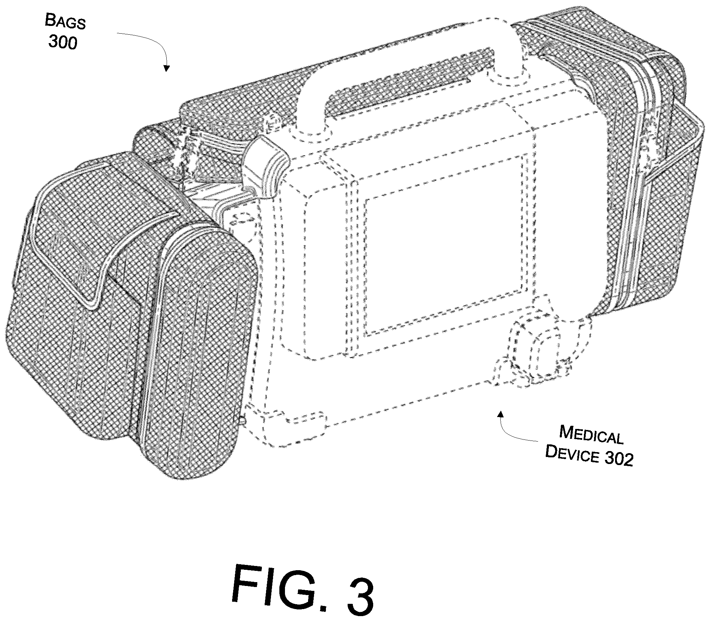

FIG. 3 illustrates an example of multiple bags 300 mounted on a medical device 302. For example, the bags 300 correspond to the bags 200, 204, 208, and 212 described above with reference to FIGS. 2A to 2D.

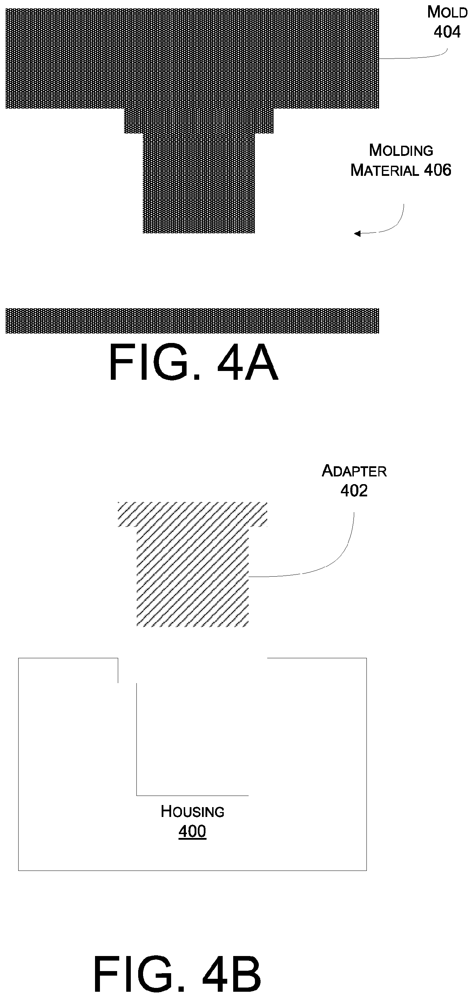

FIGS. 4A and 4B illustrate process of injection molding a housing 400 that can accommodate an adapter 402 without having an undercut. FIG. 4A illustrates a mold 404 that is configured to be injected with a molding material 406. In various cases, the mold 404 includes a concave portion that occupies a space that can accommodate the adapter 402. Because the concave portion of the mold 404 does not generate an undercut when the molding material 406 is injected into the mold 404, the mold 404 can be easily removed from the housing 400 when the molding material 406 cures or otherwise solidifies. The molding material 406, for instance, includes a metal and/or a polymer. FIG. 4B illustrates how the adapter 402 can be inserted into the housing 400. In some cases, the adapter 402 is welded (e.g., ultrasonically welded) and/or adhered to the housing 400.

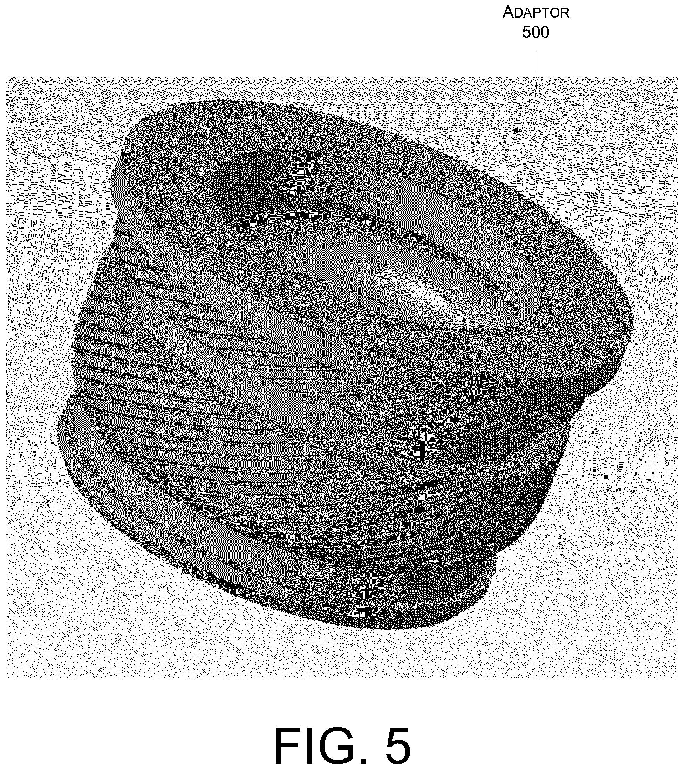

FIG. 5 illustrates an example of an adapter 500. For example, the adapter 500 can correspond to the adapter 134 and/or the adapter 402 described above. The adapter 500, in various cases, may be configured to accommodate a fastener. The fastener may be configured to be removably coupled to the adapter 500. Together, the adapter 500 and the fastener may be configured to connect a bracket, a bag, a shoulder strap, or some other apparatus, to a medical device. In various cases, the adapter 500 includes a metal, such as brass.

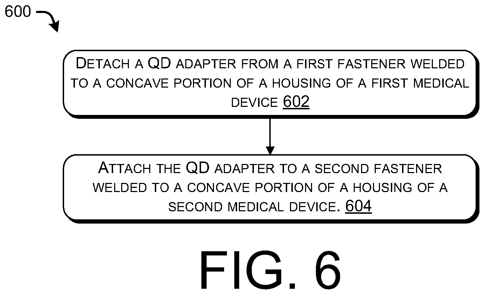

FIG. 6 illustrates an example process 600 for switching an fastener from a first medical device to a second medical device. The process 600 may be performed by an entity such as a rescuer, the fastener, an accessory, or any combination thereof.

At 602, the entity detaches a QD fastener from a first adapter that is welded to a concave portion of a housing of a first medical device. In various cases, the QD fastener is detached by retracting a protrusion of the QD fastener in an interior space of the first adapter. For instance, the first adapter is ultrasound welded to a plastic lining the concave portion of the housing. In some cases, the housing includes the plastic. The QD fastener may be physically coupled to one or more physical elements. In some cases, the QD fastener is attached to a bag or a carrying strap. In various cases, the protrusion is retracted in response to a button being pressed. In some cases, the medical device is a monitor-defibrillator, a mechanical chest compression device, a ventilation device, or some other portable medical device.

At 604, the entity attaches the QD fastener to a second fastener welded to a concave portion of a housing of a second medical device. For example, the protrusion of the QD fastener is extended into an interior space of the second adapter. In some cases, the protrusion is extended by operation of a spring inside of the QD fastener. According to some examples, the second adapter is dimensionally equivalent to the first adapter. In various examples, the second medical device is a different type of medical device than the first medical device. For example, the first medical device may be a monitor-defibrillator and the second medical device may be a mechanical chest compression device. For instance, a single shoulder strap utilizing the QD fastener can be adapted to carry multiple types of medical devices at a rescue scene.

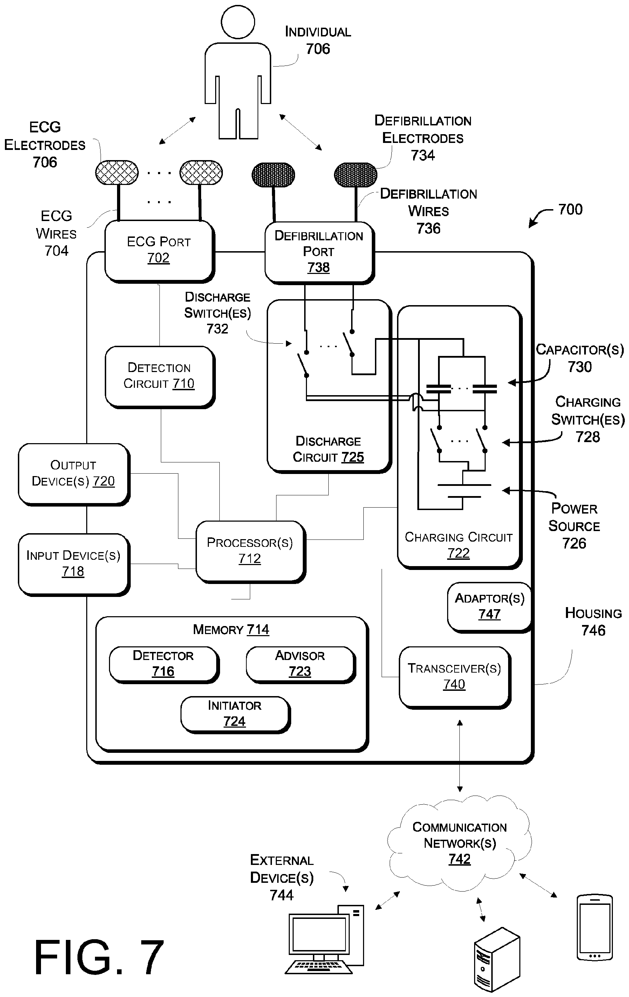

FIG. 7 illustrates an example of an external defibrillator 700 configured to perform various functions described herein. For example, the external defibrillator 700 is the medical device 102 described above with reference to FIG. 1.

The external defibrillator 700 includes an electrocardiogram (ECG) port 702 connected to multiple ECG wires 704. In some cases, the ECG wires 704 are removeable from the ECG port 702. For instance, the ECG wires 704 are plugged into the ECG port 702. The ECG wires 704 are connected to ECG electrodes 706, respectively. In various implementations, the ECG electrodes 706 are disposed on different locations on an individual 708. A detection circuit 710 is configured to detect relative voltages between the ECG electrodes 706. These voltages are indicative of the electrical activity of the heart of the individual 708.

In various implementations, the ECG electrodes 706 are in contact with the different locations on the skin of the individual 708. In some examples, a first one of the ECG electrodes 706 is placed on the skin between the heart and right arm of the individual 708, a second one of the ECG electrodes 706 is placed on the skin between the heart and left arm of the individual 708, and a third one of the ECG electrodes 706 is placed on the skin between the heart and a leg (either the left leg or the right leg) of the individual 708. In these examples, the detection circuit 710 is configured to measure the relative voltages between the first, second, and third ECG electrodes 706. Respective pairings of the ECG electrodes 706 are referred to as "leads," and the voltages between the pairs of ECG electrodes 706 are known as "lead voltages." In some examples, more than three ECG electrodes 706 are included, such that 5-lead or 12-lead ECG signals are detected by the detection circuit 710.

The detection circuit 710 includes at least one analog circuit, at least one digital circuit, or a combination thereof. The detection circuit 710 receives the analog electrical signals from the ECG electrodes 706, via the ECG port 702 and the ECG wires 704. In some cases, the detection circuit 710 includes one or more analog filters configured to filter noise and/or artifact from the electrical signals. The detection circuit 710 includes an analog-to-digital (ADC) in various examples. The detection circuit 710 generates a digital signal indicative of the analog electrical signals from the ECG electrodes 706. This digital signal can be referred to as an "ECG signal" or an "ECG."

In some cases, the detection circuit 710 further detects an electrical impedance between at least one pair of the ECG electrodes 706. For example, the detection circuit 710 includes, or otherwise controls, a power source that applies a known voltage (or current) across a pair of the ECG electrodes 706 and detects a resultant current (or voltage) between the pair of the ECG electrodes 706. The impedance is generated based on the applied signal (voltage or current) and the resultant signal (current or voltage). In various cases, the impedance corresponds to respiration of the individual 708, chest compressions performed on the individual 708, and other physiological states of the individual 708. In various examples, the detection circuit 710 includes one or more analog filters configured to filter noise and/or artifact from the resultant signal. The detection circuit 710 generates a digital signal indicative of the impedance using an ADC. This digital signal can be referred to as an "impedance signal" or an "impedance."

The detection circuit 710 provides the ECG signal and/or the impedance signal one or more processors 712 in the external defibrillator 700. In some implementations, the processor(s) 712 includes a central processing unit (CPU), a graphics processing unit (GPU), both CPU and GPU, or other processing unit or component known in the art.

The processor(s) 712 is operably connected to memory 714. In various implementations, the memory 714 is volatile (such as random access memory (RAM)), non-volatile (such as read only memory (ROM), flash memory, etc.) or some combination of the two. The memory 714 stores instructions that, when executed by the processor(s) 712, causes the processor(s) 712 to perform various operations. In various examples, the memory 714 stores methods, threads, processes, applications, objects, modules, any other sort of executable instruction, or a combination thereof. In some cases, the memory 714 stores files, databases, or a combination thereof. In some examples, the memory 714 includes, but is not limited to, RAM, ROM, electrically erasable programmable read-only memory (EEPROM), flash memory, or any other memory technology. In some examples, the memory 714 includes one or more of CD-ROMs, digital versatile discs (DVDs), content-addressable memory (CAM), or other optical storage, magnetic cassettes, magnetic tape, magnetic disk storage or other magnetic storage devices, or any other medium which can be used to store the desired information and which can be accessed by the processor(s) 712 and/or the external defibrillator 700. In some cases, the memory 714 at least temporarily stores the ECG signal and/or the impedance signal.

In various examples, the memory 714 includes a detector 716, which causes the processor(s) 712 to determine, based on the ECG signal and/or the impedance signal, whether the individual 708 is exhibiting a particular heart rhythm. For instance, the processor(s) 712 determines whether the individual 708 is experiencing a shockable rhythm that is treatable by defibrillation. Examples of shockable rhythms include ventricular fibrillation (VF) and ventricular tachycardia (V-Tach). In some examples, the processor(s) 712 determines whether any of a variety of different rhythms (e.g., asystole, sinus rhythm, atrial fibrillation (AF), etc.) are present in the ECG signal.

The processor(s) 712 is operably connected to one or more input devices 718 and one or more output devices 720. Collectively, the input device(s) 718 and the output device(s) 720 function as an interface between a user and the defibrillator 700. The input device(s) 718 is configured to receive an input from a user and includes at least one of a keypad, a cursor control, a touch-sensitive display, a voice input device (e.g., a speaker), a haptic feedback device, or any combination thereof. The output device(s) 720 includes at least one of a display, a speaker, a haptic output device, a printer, or any combination thereof. In various examples, the processor(s) 712 causes a display among the input device(s) 718 to visually output a waveform of the ECG signal and/or the impedance signal. In some implementations, the input device(s) 718 includes one or more touch sensors, the output device(s) 720 includes a display screen, and the touch sensor(s) are integrated with the display screen. Thus, in some cases, the external defibrillator 700 includes a touchscreen configured to receive user input signal(s) and visually output physiological parameters, such as the ECG signal and/or the impedance signal.

In some examples, the memory 714 includes an advisor 723, which, when executed by the processor(s) 712, causes the processor(s) 712 to generate advice and/or control the output device(s) 720 to output the advice to a user (e.g., a rescuer). In some examples, the processor(s) 712 provides, or causes the output device(s) 720 to provide, an instruction to perform CPR on the individual 708. In some cases, the processor(s) 712 evaluates, based on the ECG signal, the impedance signal, or other physiological parameters, CPR being performed on the individual 708 and causes the output device(s) 720 to provide feedback about the CPR in the instruction. According to some examples, the processor(s) 712, upon identifying that a shockable rhythm is present in the ECG signal, causes the output device(s) 720 to output an instruction and/or recommendation to administer a defibrillation shock to the individual 708.

The memory 714 also includes an initiator 724 which, when executed by the processor(s) 712, causes the processor(s) 712 to control other elements of the external defibrillator 700 in order to administer a defibrillation shock to the individual 708. In some examples, the processor(s) 712 executing the initiator 724 selectively causes the administration of the defibrillation shock based on determining that the individual 708 is exhibiting the shockable rhythm and/or based on an input from a user (received, e.g., by the input device(s) 718. In some cases, the processor(s) 712 causes the defibrillation shock to be output at a particular time, which is determined by the processor(s) 712 based on the ECG signal and/or the impedance signal.

The processor(s) 712 is operably connected to a charging circuit 722 and a discharge circuit 725. In various implementations, the charging circuit 722 includes a power source 726, one or more charging switches 728, and one or more capacitors 730. The power source 726 includes, for instance, a battery. The processor(s) 712 initiates a defibrillation shock by causing the power source 726 to charge at least one capacitor among the capacitor(s) 730. For example, the processor(s) 712 activates at least one of the charging switch(es) 728 in the charging circuit 722 to complete a first circuit connecting the power source 726 and the capacitor to be charged. Then, the processor(s) 712 causes the discharge circuit 725 to discharge energy stored in the charged capacitor across a pair of defibrillation electrodes 734, which are in contact with the individual 708. For example, the processor(s) 712 deactivates the charging switch(es) 728 completing the first circuit between the capacitor(s) 730 and the power source 726, and activates one or more discharge switches 732 completing a second circuit connecting the charged capacitor 730 and at least a portion of the individual 708 disposed between defibrillation electrodes 734.

The energy is discharged from the defibrillation electrodes 734 in the form of a defibrillation shock. For example, the defibrillation electrodes 734 are connected to the skin of the individual 708 and located at positions on different sides of the heart of the individual 708, such that the defibrillation shock is applied across the heart of the individual 708. The defibrillation shock, in various examples, depolarizes a significant number of heart cells in a short amount of time. The defibrillation shock, for example, interrupts the propagation of the shockable rhythm (e.g., VF or V-Tach) through the heart. In some examples, the defibrillation shock is 200 J or greater with a duration of about 0.015 seconds. In some cases, the defibrillation shock has a multiphasic (e.g., biphasic) waveform. The discharge switch(es) 732 are controlled by the processor(s) 712, for example. In various implementations, the defibrillation electrodes 734 are connected to defibrillation wires 736. The defibrillation wires 736 are connected to a defibrillation port 738, in implementations. According to various examples, the defibrillation wires 736 are removable from the defibrillation port 738. For example, the defibrillation wires 736 are plugged into the defibrillation port 738.

In various implementations, the processor(s) 712 is operably connected to one or more transceivers 740 that transmit and/or receive data over one or more communication networks 742. For example, the transceiver(s) 740 includes a network interface card (NIC), a network adapter, a local area network (LAN) adapter, or a physical, virtual, or logical address to connect to the various external devices and/or systems. In various examples, the transceiver(s) 740 includes any sort of wireless transceivers capable of engaging in wireless communication (e.g., radio frequency (RF) communication). For example, the communication network(s) 742 includes one or more wireless networks that include a 3^{rd} Generation Partnership Project (3GPP) network, such as a Long Term Evolution (LTE) radio access network (RAN) (e.g., over one or more LE bands), a New Radio (NR) RAN (e.g., over one or more NR bands), or a combination thereof. In some cases, the transceiver(s) 740 includes other wireless modems, such as a modem for engaging in WI-FI^{®}, WIGIG^{®}, WIMAX^{®}, BLUETOOTH^{®}, or infrared communication over the communication network(s) 742.

The defibrillator 700 is configured to transmit and/or receive data (e.g., ECG data, impedance data, data indicative of one or more detected heart rhythms of the individual 708, data indicative of one or more defibrillation shocks administered to the individual 708, etc.) with one or more external devices 744 via the communication network(s) 742. The external devices 744 include, for instance, mobile devices (e.g., mobile phones, smart watches, etc.), Internet of Things (IoT) devices, medical devices, computers (e.g., laptop devices, servers, etc.), or any other type of computing device configured to communicate over the communication network(s) 742. In some examples, the external device(s) 744 is located remotely from the defibrillator 700, such as at a remote clinical environment (e.g., a hospital). According to various implementations, the processor(s) 712 causes the transceiver(s) 740 to transmit data to the external device(s) 744. In some cases, the transceiver(s) 740 receives data from the external device(s) 744 and the transceiver(s) 740 provide the received data to the processor(s) 712 for further analysis.

In various implementations, the external defibrillator 700 also includes a housing 746 that at least partially encloses other elements of the external defibrillator 700. For example, the housing 746 encloses the detection circuit 710, the processor(s) 712, the memory 714, the charging circuit 722, the transceiver(s) 740, or any combination thereof. In some cases, the input device(s) 718 and output device(s) 720 extend from an interior space at least partially surrounded by the housing 746 through a wall of the housing 746. In various examples, the housing 746 acts as a barrier to moisture, electrical interference, and/or dust, thereby protecting various components in the external defibrillator 700 from damage. In various implementations, one or more adapters 747 are disposed in the housing 746.

In some implementations, the external defibrillator 700 is an automated external defibrillator (AED) operated by an untrained user (e.g., a bystander, layperson, etc.) and can be operated in an automatic mode. In automatic mode, the processor(s) 712 automatically identifies a rhythm in the ECG signal, makes a decision whether to administer a defibrillation shock, charges the capacitor(s) 730, discharges the capacitor(s) 730, or any combination thereof. In some cases, the processor(s) 712 controls the output device(s) 720 to output (e.g., display) a simplified user interface to the untrained user. For example, the processor(s) 712 refrains from causing the output device(s) 720 to display a waveform of the ECG signal and/or the impedance signal to the untrained user, in order to simplify operation of the external defibrillator 700.

In some examples, the external defibrillator 700 is a monitor-defibrillator utilized by a trained user (e.g., a clinician, an emergency responder, etc.) and can be operated in a manual mode or the automatic mode. When the external defibrillator 700 operates in manual mode, the processor(s) 712 cause the output device(s) 720 to display a variety of information that may be relevant to the trained user, such as waveforms indicating the ECG data and/or impedance data, notifications about detected heart rhythms, and the like.

### EXAMPLE CLAUSES

1. A portable medical device system, the system including: a medical device including: a housing; and an accessory port disposed in the housing; a bracket removably coupled to the housing of the medical device, the bracket including: a first arm disposed on a vertical side of the housing; a second arm disposed on a bottom side of the housing; and a foot extending from the second arm, the foot including a compressible material with a coefficient of friction that is greater than a threshold coefficient of friction; an accessory bag removably coupled to the first arm of the bracket, the accessory bag including: a rigid compartment enclosing an interior space; a zipper extending along a segment of the rigid compartment, the segment being within a threshold distance of the accessory port; and a flap physically coupled to an exterior of the rigid compartment and configured to hang over the zipper extending along the segment of the rigid compartment; and an accessory device including: a plug configured to be electrically coupled to the accessory port; an accessory configured to detect a physiological parameter or to administer a treatment, the accessory being disposed in the interior space of the rigid compartment; and a wire extending: between the plug and the accessory; and through the segment of the rigid compartment.
2. The portable medical device system of clause 1, wherein the first arm and the second arm of the bracket include a plastic or metal, and wherein the bracket is removably coupled to the housing of the medical device by multiple fasteners.
3. The portable medical device system of clause 1 or 2, the bracket being a first bracket, the vertical side being a first vertical side, the accessory bag being a first accessory bag, the accessory device being a first accessory device, the portable medical device system further including: a second bracket removably coupled to a second vertical side of the housing of the medical device and to the bottom side of the medical device; a second accessory bag removably coupled to the second bracket; a third bracket removably coupled to a third vertical side of the housing of the medical device; a first storage bag coupled to the third bracket; a second storage bag removably coupled to an upper surface of the medical device; and a shoulder strap removably coupled to the first bracket and to the second bracket.
4. A modular bag system for a medical device, the modular bag system including:
   a bracket configured to be removably coupled to a medical device housing, the bracket including: a first arm extending in a first direction and configured to conform to a portion of a first side of the medical device housing; a second arm extending in a second direction and
   configured to conform to a portion of a second side of the medical device housing; a foot extending from the second arm; and a bag removably coupled to the first arm of the bracket.
5. The modular bag system of clause 4, wherein the first arm and the second arm include a rigid material including a plastic or a metal, and wherein the foot includes a rubberized material.
6. The modular bag system of clause 4 or 5, wherein a hole extends through the first arm, the hole being configured to accommodate a fastener of a shoulder strap.
7. The modular bag system of any of clauses 4 to 6, wherein holes extend through the second arm, the holes being configured to accommodate fasteners.
8. The modular bag system of any of clauses 4 to 7, wherein the bracket further includes a connecting piece physically coupled to the first arm and to the second arm.
9. The modular bag system of any of clauses 4 to 8, wherein the bag includes: a rigid compartment enclosing a storage space.
10. The modular bag system of clause 9, wherein the bag further includes: a zipper extending along a first segment of the rigid compartment; and a flap coupled to a second segment of the rigid compartment, the second segment being parallel to the first segment along the rigid compartment.
11. The modular bag system of clause 10, wherein the flap includes a woven material.
12. The modular bag system of any of clauses 4 to 11, the bracket being a first bracket, the portion of the second side of the medical device housing being a first portion of the second side of the medical device housing, the foot being a first foot, the bag being a first bag, the modular bag system further including: a second bracket configured to be removably coupled to the medical device housing, the second bracket including: a third arm extending in the first direction and configured to conform to a portion of a third side of the medical device housing; a fourth arm extending in the second direction and configured to conform to a second portion of the second side of the medical device housing; and a second foot extending from the fourth arm; and a second bag removably coupled to the third arm of the bracket.
13. The modular bag system of clause 12, wherein the first arm is further configured to conform to a first corner of the medical device housing, and wherein the third arm is further configured to confirm to a second corner of the medical device housing.
14. The modular bag system of clause 12 or 13, further including: a strap removably coupled to the first arm and the third arm.
15. The modular bag system of any of clauses 4 to 14, the bracket being a first bracket, the bag being a first bag, the modular bag system further including: a second bracket configured to be removably coupled to a portion of a third side of the medical device housing; and a second bag physically coupled to the second bracket.
16. The modular bag system of any of clauses 4 to 15, the bag being a first bag, the modular bag system further including: a second bag configured to be removably coupled to a third side of the medical device housing.
17. A portable medical device, including: a circuit configured to detect a physiological parameter of a subject or to output a treatment signal to the subject; a power source electrically coupled to the circuit; a housing including a plastic and enclosing the circuit and the power source; a first fastener including brass and being ultrasound-welded to the plastic of the housing, a first surface of the first fastener being coplanar with an outer surface of the housing, a second surface of the first fastener extending perpendicularly from the first surface of the first fastener, a third surface of the first fastener extending from the second surface of the first surface and having a curved shape, a second fastener disposed in the housing; and a shoulder strap including: a first quick-detach (QD) sling swivel including: protrusions extending into a space defined by the third surface of the first fastener when the first QD sling swivel is removably coupled to the first fastener; and a button that, when pressed, causes the protrusions to retract; a second QD sling swivel configured to be removably coupled to the second fastener; and a band extending between the first QD sling swivel and the second QD sling swivel.
18. The portable medical device of clause 17, wherein the first fastener is ultrasound-welded to a concave portion of the plastic of the housing, the concave portion lacking an undercut, and wherein the first fastener is directly in contact with the plastic of the housing.
19. The portable medical device of clause 17 or 18, wherein the first fastener is dimensionally equivalent to a third fastener that is disposed in a housing of another portable medical device.
20. A portable medical device, including: a housing; and an adapter including a metal and being ultrasound-welded to the housing, a first surface of the adapter being coplanar with an outer surface of the housing, a second surface of the adapter extending perpendicularly from the first surface of the adapter and having a narrow radius, a third surface of the adapter extending from the second surface of the first surface and having a greater radius than the narrow radius.
21. The portable medical device of clause 20, wherein the housing includes a plastic, and wherein the adapter is welded directly to the plastic of the housing.
22. The portable medical device of clause 20 or 21, wherein the metal includes brass.
23. The portable medical device of any of clauses 20 to 22, wherein the adapter is disposed in a concave portion in the outer surface of the housing, the concave portion lacking an undercut.
24. The portable medical device of any of clauses 20 to 23, wherein the portable medical device includes a monitor-defibrillator, an accessory device, a patient monitor, a ventilation device, or a mechanical chest compression device.
25. The portable medical device of any of clauses 20 to 24, further including: a carrying strap including: a QD fastener configured to be inserted into the adapter, the QD fastener including a protrusion configured to extend to the third surface; and a band extending from the QD fastener.
26. The portable medical device of any of clauses 20 to 25, further including: a bag including: a rigid compartment enclosing an interior space; and a QD fastener coupled to the rigid compartment, the QD fastener being configured to be inserted into the adapter and including a protrusion configured to extend to the third surface.
27. A method, including: detaching a QD fastener from a first adapter welded to a concave portion of a housing of a first portable medical device by retracting a protrusion of the QD fastener in an interior space of the first adapter; and attaching the QD fastener to a second adapter welded to a concave portion of a housing of a second portable medical device by extending the protrusion of the QD fastener in an interior space of the second adapter.
28. The method of clause 27, wherein the first fastener is ultrasound welded to a plastic lining the concave portion of the housing of the first portable medical device, and wherein the second adapter is ultrasound-welded to a plastic lining the concave portion of the housing of the second portable medical device.
29. The method of clause 27 or 28, wherein the QD fastener is physically coupled to a bag or a carrying strap.
30. The method of any of clauses 27 to 29, wherein retracting the protrusion of the QD fastener in the interior space of the first adapter includes: receiving, by a button of the QD fastener, a press from a user.
31. The method of any of clauses 27 to 30, wherein the first portable medical device or the second portable medical device includes a monitor-defibrillator.
32. The method of any of clauses 27 to 31, wherein the first portable medical device or the second portable medical device includes a mechanical chest compression device.
33. The method of any of clauses 27 to 32, wherein extending the protrusion of the QD fastener in the interior space of the second adapter is performed by a spring.
34. The method of any of clauses 27 to 33, wherein retracting the protrusion of the QD fastener in the interior space of the first adapter includes reducing a width of an inserted portion of the QD fastener to a distance that is smaller than a radius of an opening of the first adapter.
35. The method of any of clauses 27 to 34, wherein extending the protrusion of the QD fastener in the interior space of the second adapter includes increasing a width of an inserted portion of the QD fastener to a distance that is greater than a radius of an opening of the second adapter.
36. The method of any of clauses 27 to 35, wherein the first adapter is dimensionally equivalent to the second adapter.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be used for realizing implementations of the disclosure in diverse forms thereof.

As will be understood by one of ordinary skill in the art, each implementation disclosed herein can comprise, consist essentially of or consist of its particular stated element, step, or component. Thus, the terms "include" or "including" should be interpreted to recite: "comprise, consist of, or consist essentially of." The transition term "comprise" or "comprises" means has, but is not limited to, and allows for the inclusion of unspecified elements, steps, ingredients, or components, even in major amounts. The transitional phrase "consisting of" excludes any element, step, ingredient or component not specified. The transition phrase "consisting essentially of' limits the scope of the implementation to the specified elements, steps, ingredients or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any non-claimed element essential to the practice of implementations of the disclosure.

Groupings of alternative elements or implementations disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain implementations are described herein, including the best mode known to the inventors for carrying out implementations of the disclosure. Of course, variations on these described implementations will become apparent to those of ordinary skill in the art upon reading the foregoing description. The inventors expect skilled artisans to employ such variations as appropriate, and the inventors intend for implementations to be practiced otherwise than specifically described herein. Accordingly, the scope of this disclosure includes all modifications and equivalents of the subject matter recited in the claims appended hereto as permitted by applicable law. Moreover, any combination of the above-described elements in all possible variations thereof is encompassed by implementations of the disclosure unless otherwise indicated herein or otherwise clearly contradicted by context.

## Claims

1. A modular bag system for a medical device, the modular bag system comprising:
a bracket configured to be removably coupled to a medical device housing, the bracket comprising:
a first arm extending in a first direction and configured to conform to a portion of a first side of the medical device housing;
a second arm extending in a second direction and configured to conform to a portion of a second side of the medical device housing;
a foot extending from the second arm; and
a bag removably coupled to the first arm of the bracket.

2. The modular bag system of claim 1, wherein the first arm and the second arm comprise a rigid material comprising a plastic or a metal, and
wherein the foot comprises a rubberized material.

3. The modular bag system of claim 1 or 2, wherein one or more holez extends through the first arm, the one or more holes being configured to accommodate a fastener, such as of a shoulder strap.

4. The modular bag system of claim 1, 2 or 3, wherein the bracket further comprises a connecting piece physically coupled to the first arm and to the second arm.

5. The modular bag system of any of claims 1-4, wherein the bag comprises:
a rigid compartment enclosing a storage space.

6. The modular bag system of claim 5, wherein the bag further comprises:
a zipper extending along a first segment of the rigid compartment; and
a flap coupled to a second segment of the rigid compartment, the second segment being parallel to the first segment along the rigid compartment.

7. The modular bag system of claim 6, wherein the flap comprises a woven material.

8. The modular bag system of any of claims 1-7, the bracket being a first bracket, the portion of the second side of the medical device housing being a first portion of the second side of the medical device housing, the foot being a first foot, the bag being a first bag, the modular bag system further comprising:
a second bracket configured to be removably coupled to the medical device housing, the second bracket comprising:
a third arm extending in the first direction and configured to conform to a portion of a third side of the medical device housing;
a fourth arm extending in the second direction and configured to conform to a second portion of the second side of the medical device housing; and
a second foot extending from the fourth arm; and
a second bag removably coupled to the third arm of the bracket.

9. The modular bag system of claim 8, wherein the first arm is further configured to conform to a first corner of the medical device housing, and
wherein the third arm is further configured to confirm to a second corner of the medical device housing.

10. The modular bag system of claim 8, further comprising:
a strap removably coupled to the first arm and the third arm.

11. The modular bag system of any of claims 1-10, the bracket being a first bracket, the bag being a first bag, the modular bag system further comprising:
a second bracket configured to be removably coupled to a portion of a third side of the medical device housing; and
a second bag physically coupled to the second bracket.

12. The modular bag system of any of claims 1-11, the bag being a first bag, the modular bag system further comprising:
a second bag configured to be removably coupled to a third side of the medical device housing.

13. A portable medical device system, the system comprising:
a medical device comprising:
a housing; and
an accessory port disposed in the housing;
a bracket removably coupled to the housing of the medical device, the bracket comprising:
a first arm disposed on a vertical side of the housing;
a second arm disposed on a bottom side of the housing; and
a foot extending from the second arm, the foot comprising a compressible material with a coefficient of friction that is greater than a threshold coefficient of friction;
an accessory bag removably coupled to the first arm of the bracket, the accessory bag comprising:
a rigid compartment enclosing an interior space;
a zipper extending along a segment of the rigid compartment, the segment being within a threshold distance of the accessory port; and
a flap physically coupled to an exterior of the rigid compartment and configured to hang over the zipper extending along the segment of the rigid compartment; and an accessory device comprising:
a plug configured to be electrically coupled to the accessory port;
an accessory configured to detect a physiological parameter or to administer a treatment, the accessory being disposed in the interior space of the rigid compartment; and
a wire extending:
between the plug and the accessory; and
through the segment of the rigid compartment.

14. The portable medical device system of claim 13, wherein the first arm and the second arm of the bracket comprise a plastic or metal, and
wherein the bracket is removably coupled to the housing of the medical device by multiple fasteners.

15. The portable medical device system of claim 13 or 14, the bracket being a first bracket, the vertical side being a first vertical side, the accessory bag being a first accessory bag, the accessory device being a first accessory device, the portable medical device system further comprising:
a second bracket removably coupled to a second vertical side of the housing of the medical device and to the bottom side of the medical device;
a second accessory bag removably coupled to the second bracket;
a third bracket removably coupled to a third vertical side of the housing of the medical device;
a first storage bag coupled to the third bracket;
a second storage bag removably coupled to an upper surface of the medical device; and
a shoulder strap removably coupled to the first bracket and to the second bracket.
